(19) Europäisches Patentamt
European Patent Office
Office européen des brevets

(11) Veröffentlichungsnummer: **0 198 311**
**A2**

(12) **EUROPÄISCHE PATENTANMELDUNG**

(21) Anmeldenummer: **86104415.4**

(51) Int. Cl.⁴: **C07D 213/82**, A01N 43/40

(22) Anmeldetag: **01.04.86**

(30) Priorität: **04.04.85 CH 1468/85**
**09.01.86 CH 38/86**

(43) Veröffentlichungstag der Anmeldung:
**22.10.86 Patentblatt 86/43**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Isenring, Hans-Peter, Dr.**
**Himmelrainweg 5**
**CH-4450 Sissach(CH)**
Erfinder: **Ziegler, Hugo, Dr.**
**Riburgstrasse 8**
**CH-4058 Basel(CH)**

(74) Vertreter: **Lederer, Franz, Dr. et al**
**Vanderwerth, Lederer & Riederer**
**Patentanwälte Lucile-Grahn-Strasse 22**
**D-8000 München 80(DE)**

(54) **Nicotinamide und deren Verwendung als fungizide Wirkstoffe.**

(57) Die Erfindung betrifft neue Nicotinamide der

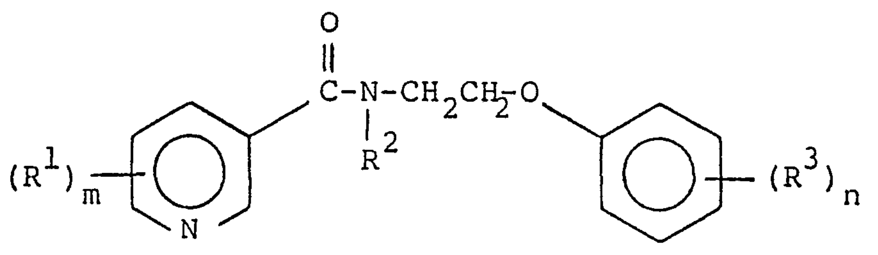

I

Formel worin $R^1$, $R^2$, $R^3$, m und n die in der Beschreibung angegebenen Bedeutungen besitzen, und ihre Säureadditionssalze, Verfahren zu deren Herstellung, fungizide Mittel, die diese Verbindungen als Wirkstoffe enthalten, sowie die Verwendung solcher Verbindungen bzw. Mittel zur Bekämpfung von Fungi in der Landwirtschaft und im Gartenbau.

EP 0 198 311 A2

Nicotinamide und deren Verwendung als fungizide Wirkstoffe

Die vorliegende Erfindung betrifft heterocyclische Verbindungen, und zwar Nicotinamide der allgemeinen Formel

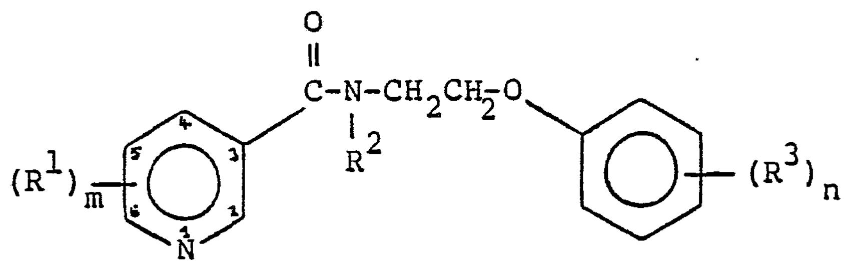

I

worin

$R^1$ Halogen in der 5-und/oder 6-Stellung der Pyridylgruppe,

$R^2$ $C_{1-6}$-Alkyl, $C_{1-6}$ -Alkoxy-$C_{1-4}$-alkyl, Cyano-$C_{1-4}$-alkyl, $C_{3-6}$-Alkinyl oder gegebenenfalls im Benzolkern mono-, di-oder trisubstituiertes Benzyl, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Trifluormethylgruppe und/oder eine Nitrogruppe sind,

$R^3$ Halogen, $C_{1-3}$-Alkyl oder Nitro,

m 1 oder 2

und

n 1 , 2 oder 3 bedeuten,

und Säureadditionssalze dieser Verbindungen.

Die Verbindungen der Formel I und deren Säureadditionssalze besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der Verbindungen der Formel I und ihrer Säureadditionssalze, fungizide Mittel, die Verbindungen der Formel I oder Säureadditionssalze davon als Wirkstoffe enthalten, sowie die Verwendung solcher Verbindungen, Säureadditionssalze und Mittel zur Bekämpfung von Fungi in der Landwirtschaft und im Gartenbau.

In der obigen Formel I umfasst der Ausdruck "Halogen" Fluor, Chlor, Brom und Jod. Die Alkyl-und Alkinylgruppen können geradkettiges oder verzweigt sein, wobei dies auch für den bzw. jeden Alkylteil der Alkoxyalkyl-, Alkoxy-und Cyanoalkylgruppen gilt. In der disubstituierten 3-Pyridylgruppe oder der di-oder trisubstituierten Phenylgruppe können die Substituenten $R^1$ bzw. $R^3$ gleich oder verschieden sein. Auch im Falle, dass $R^2$ di-oder trisubstituiertes Benzyl bedeutet, können die Substituenten gleich oder verschieden sein.

Das Vorhandensein mindestens eines asymmetrischen Kohlenstoffatoms in den Verbindungen der Formel I hat zur Folge, dass die Verbindungen in optisch isomeren Formen auftreten können. Unabhängig davon kommt auch in gewissen Fällen eine Atropisomerie vor. Die Formel I soll all diese möglichen isomeren Formen umfassen.

Als Säureadditionssalze der Verbindungen der Formel I kommen physiologisch verträgliche Salze in Frage. Hierzu ge hören vorzugsweise Salze dieser Verbindungen mit anorganischen und organischen Säuren, wie Salzsäure; Salpetersäure; Phosphorsäure; mono-und bifunktionellen Carbonsäuren und Hydroxycarbonsäuren, z.B. Essigsäure, Maleinsäure, Bernsteinsäure, Fumarsäure, Weinsäure, Zitronensäure, Salicylsäure, Sorbinsäure und Milchsäure; und Sulfonsäuren, z.B. 1,5-Naphthalin-disulfonsäure.

Was die bevorzugten Bedeutungen von $R^1$, $R^2$, $R^3$, m und n anbelangt, so ist folgendes zu sagen: Falls $R^2$ Alkoxyalkyl oder Cyanoalkyl bedeutet, ist dies vorzugsweise 2-Methoxyäthyl bzw. Cyanomethyl. $(R^1)m$ bedeutet vorzugsweise ein einzelnes Halogenatom in 5-Stellung der Pyridylgruppe. Ebenfalls bevorzugt befinden sich die Substituenten $R^3$ in den ortho-und/oder para-Stellungen der Phenylgruppe. Unabhängig voneinander bedeuten $R^1$ vorzugsweise Fluor, Chlor oder Brom, $R^2$ vor-

zugsweise $C_{1-6}$-Alkyl, $C_{1-6}$-Alkoxy-$C_{1-4}$ -alkyl oder gegebenenfalls substituiertes Benzyl, wie dies oben näher definiert ist, insobesondere Aethyl, n-Propyl oder n-Butyl, $R^3$ vorzugsweise Halogen oder $C_{1-3}$-Alkyl, insbesondere Chlor oder Methyl, m vorzugsweise 1 und n vorzugsweise 2 oder 3. Ganz besonders bevorzugte mit $(R^3)n$-substituierte Phenylgruppen sind 2,4,6-Trichlorphenyl, 2,4-Dichlor-6-methylphenyl und 2,6-Dichlorphenyl.

Besonders bevorzugte Verbindungen der Formel I sind:

5-Fluor-N-(n-propyl)-N-[2-(2,4,6-trichlorphenoxy)-äthyl]-nicotinamid,

5-Chlor-N-(n-propyl)-N-[2-(2,4,6-trichlorphenoxy)-äthyl]-nicotinamid,

5-Brom-N-(n-propyl)-N-[2-(2,4,6-trichlorphenoxy)-äthyl]-nicotinamid,

5-Brom-N-[2-(2,6-dichlorphenoxy)äthyl]-N-(n-propyl)-nicotinamid und

5-Brom-N-[-2-(4,6-dichlor-o-tolyloxy)äthyl]-N-(n-propyl)-nicotinamid.

Weitere Vertreter von Verbindungen der Formel I sind:

5-Fluor-N-(4-chlorbenzyl-N-[2-(2,4,6-trichlorphenoxy)äthyl]-nicotinamid,

5-Chlor-N-(4-chlorbenzyl)-N-[2-(2,4,6-trichlorphenoxy)äthyl]-nicotinamid,

5-Brom-N-(4-chlorbenzyl)-N-[2-(2,4,6-trichlorphenoxy)äthyl]-nicotinamid,

5-Fluor-N-(2,4-dichlorbenzyl)-N-[2-(2,4,6-trichlorphenoxy)äthyl]-nicotinamid,

5-Chlor-N-(2,4-dichlorbenzyl-N-[2-(2,4,6-trichlorphenoxy)äthyl]-nicotinamid,

5-Fluor-N-[2-(2,4,6-trichlorphenoxy)äthyl]-N-(4-trifluormethylbenzyl)-nicotinamid,

5-Chlor-N-[2-(2,4,6-trichlorphenoxy)äthyl]-N-(4-trifluormethylbenzyl)-nicotinamid,

5-Brom-N-[2-(2,4,6-trichlorphenoxy)äthyl]-N-(4-trifluormethylbenzyl)-nicotinamid,

5-Fluor-N-(4-methoxybenzyl)-N-[2-(2,4,6-trichlorphenoxy)äthyl]-nicotinamid,

5-Chlor-N-(4-methoxybenzyl)-N-[2-(2,4,6-trichlorphenoxy)äthyl]-nicotinamid,

5-Brom-N-(4-methoxybenzyl)-N-[2-(2,4,6-trichlorphenoxy)äthyl]-nicotinamid,

5-Fluor-N-(4-methylbenzyl)-N-[2-(2,4,6-trichlorphenoxy)äthyl]-nicotinamid,

5-Chlor-N-(4-methylbenzyl)-N-[2-(2,4,6-trichlorphenoxy)äthyl]-nicotinamid,

5-Brom-N-(4-methylbenzyl)-N-[2-(2,4,6-trichlorphenoxy)äthyl]-nicotinamid,

5-Fluor-N-(3,4-dichlorbenzyl)-N-[2-(2,4,6-trichlorphenoxy)äthyl]-nicotinamid,

5-Chlor-N-(3,4-dichlorbenzyl)-N-[2-(2,4,6-trichlorphenoxy)äthyl]-nicotinamid,

5-Brom-N-(3,4-dichlorbenzyl)-N-[2-(2,4,6-trichlorphenoxy)äthyl]-nicotinamid,

5-Fluor-N-(4-nitrobenzyl)-N-[2-(2,4,6-trichlorphenoxy)äthyl]-nicotinamid,

5-Chlor-N-(4-nitrobenzyl)-N-[2-(2,4,6-trichlorphenoxy)äthyl]-nicotinamid,

5-Brom-N-(4-nitrobenzyl)-N-[2-(2,4,6-trichlorphenoxy)äthyl]-nicotinamid,

5-Jod-N-(n-butyl)-N-[2-(2,4,6-trichlorphenoxy)äthyl]-nicotinamid,

5-Jod-N-(n-pentyl)-N-[2-(2,4,6-trichlorphenoxy)-äthyl]-nicotinamid,

5-Jod-N-äthyl-N-[2-(4,6-dichlor-o-tolyloxy)äthyl]-nicotinamid und

5-Jod-N-äthyl-N-[2-(2,6-dichlorphenoxy)äthyl]-nicotinamid.

Das erfindungsgemässe Verfahren zur Herstellung der Verbindungen der Formel I und ihrer Säureadditionssalze ist dadurch gekennzeichnet, dass man

a) ein reaktionsfähiges Derivat einer Nicotinsäure der allgemeinen Formel

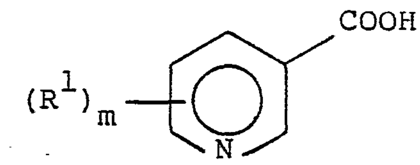

II

worin R¹ und m die oben angegebenen Bedeutungen besitzen,

mit einem 2-Phenoxyäthylamin der allgemeinen Formel

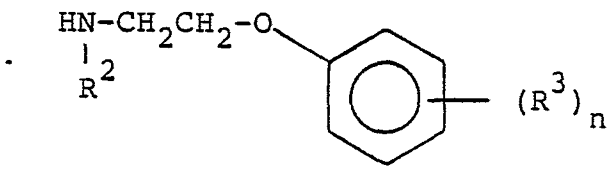

III

worin R², R³ und n die oben angegebenen Bedeutungen besitzen,

umsetzt oder

b) ein N-(2-Hydroxyäthyl)-nicotinamid der allgemeinen Formel

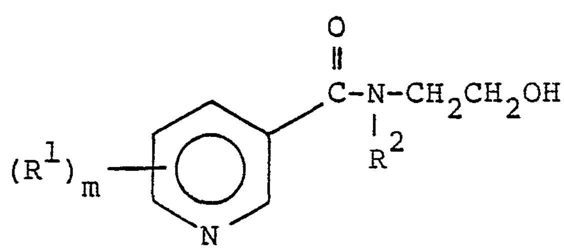

IV

worin R¹, R² und m die oben angegebenen Bedeutungen besitzen,

mit einem Phenol der allgemeinen Formel

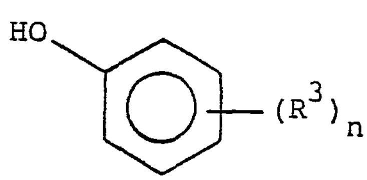

V

worin R³ und n die oben angegebenen Bedeutungen besitzen,

umsetzt,

und gewünschtenfalls eine so erhaltene Verbindung der Formel I durch Umsetzung mit einer Säure in das entsprechende Säureadditionssalz überführt.

Bei der Verfahrensvariante a) dient als reaktionsfähiges Derivat der Nicotinsäure der Formel III vorzugsweise ein Säurehalogenid, insbesondere das Säurechlorid oder Säurebromid, oder das Imidazolid. In beiden Fällen erfolgt die Umsetzung zweckmässigerweise in Gegenwart eines im wesentlichen wasserfreien, inerten Verdünnungsmittels, insbesondere eines organischen Lösungsmittels, wie eines aliphatischen oder cyclischen Aethers, z.B. 1,2-Dimethoxyäthan, tert.Butylmethyläther, Tetrahydrofuran oder Dioxan; eines Aromaten, z.B. Benzol oder Toluol; eines aliphatischen halogenierten Kohlenwasserstoffs, z.B. Methylenchlorid oder Chloroform; oder Dimethylformamid, oder eines Gemisches solcher Verdünnungsmittel bei Temperaturen zwischen ca. -20°C und ca. 150°C. Im Falle der Verwendung des Säurehalogenids wird vorzugsweise bei Temperaturen zwischen 0°C und der Rückflusstemperatur des Reaktionsgemisches, ganz bevorzugt bei Raumtemperatur, und im Falle der Verwendung des Imidazolids vorzugsweise bei Temperaturen zwischen der Raumtemperatur und ca. 120°C, ganz bevorzugt bei der Rückflusstemperatur des Reaktionsgemisches, gearbeitet.

Zudem wird die Umsetzung des Säurehalogenids mit dem 2-Phenoxyäthylamin zweckmässigerweise in Gegenwart eines organischen oder anorganischen säurebindenen Mittels durchgeführt. Als geeignete säurebindende Mittel kommen insbesondere tertiäre Amine, wie Triäthylamin und Pyridin, sowie Alkalimetallcarbonate und -hydrogencarbonate, wie Natrium-und Kaliumcarbonat und Natrium-und Kaliumhydrogencarbonat, in Frage.

Bei der Verwendung des Imidazolids wird dieses geeigneterweise in situ erzeugt, indem man die Nicotinsäure der Formel II mit Carbonyldiimidazol in Gegenwart eines im wesentlichen wasserfreien, inerten Verdünnungsmittels, insbesondere eines der oben erwähnten organischen Lösungsmittel, und bei erhöhter Temperatur, insbesondere bei der Rückflusstemperatur des Reaktionsgemisches, umsetzt. Vorzugsweise werden die Reaktionsteilnehmer in im wesentlichen äquimolaren Mengen miteinander umgesetzt. Anschliessend wird eventuell nach Kühlung des Reaktionsgemisches auf Raumtemperatur das 2-Phenoxyäthylamin der Formel III zum Gemisch gegeben, und zwar ebenfalls vorzugsweise in äquimolarer Menge, und die Umsetzung des in situ erzeugten Imidazolids mit dem Amin wird dann wie oben beschrieben in demselben Reaktionsmedium durchgeführt. Das Imidazolid kann jedoch zunächst in herkömmlicher Weise isoliert werden und mit dem Amin in erneutem Verdünnungsmittel umgesetzt werden.

Die Verfahrensvariante b) wird zweckmässigerweise unter Verwendung des an sich bekannten Redox-Reaktionssystems Azodicarbonsäureester/Triphenylphosphin in Gegenwart eines im wesentlichen wasserfreien inerten Verdünnungsmittels durchgeführt. Als Verdünnungsmittel eignen sich insbesondere aprotische organische Lösungsmittel, wie aliphatische oder cyclische Aether, z.B. Diäthyläther, 1,2-Dimethoxyäthan, Tetrahydrofuran und Dioxan; Aromaten, z.B. Benzol und Toluol; halogenierte Kohlenwasserstoffe, z.B. Methylenchlorid; und Hexamethylphosphorsäuretriamid. Die Reaktionstemperaturen liegen zweckmässigerweise zwischen ca. 0°C und 60°C, wobei ganz bevorzugt bei Raumtemperatur gearbeitet wird. Gemäss der an sich bekannten Arbeitsweise wird entweder der Azodicarbonsäureester zu einer Lösung von Triphenylphosphin und den Ausgangsmaterialien der Formeln IV und V bei Raumtemperatur oder einer niedrigeren Temperatur, oder das Triphenylphosphin zu den anderen drei Reaktionsteilnehmern gegeben, wobei die ersterwähnte Reihenfolge die bevorzugte ist. Der Azocarbonsäureester ist vorzugsweise der Diäthyl-oder Diisopropylester

Zur Herstellung der Säureadditionssalze der Verbindungen der Formel I werden diese mit den gewünschten Säuren auf übliche Weise umgesetzt.

Die Isolierung und Reinigung der so hergestellten Verbindungen der Formel I bzw. der Säureadditionssalze können nach an sich bekannten Methoden erfolgen. Zudem können gegebenenfalls anfallende Isomerengemische dieser Verbindungen nach an sich bekannten Methoden in die einzelnen Isomeren aufgetrennt werden.

Die in der Verfahrensvariante b) benötigten Ausgangsmaterialien der Formel IV sind neu und können dadurch hergestellt werden, dass man ein reaktionsfähiges Derivat einer Nicotinsäure der oben angegebenen Formel II mit einem Aethanolamin der allgemeinen Formel

$R^2HN\text{-}CH_2CH_2\text{-}OH$ VI

worin $R^2$ die oben angegebene Bedeutung besitzt, umsetzt, und zwar unter den gleichen Reaktionsbedingungen, wie diese oben im Zusammenhang mit der Verfahrensvariante a) beschrieben sind. Als weitere Herstellungsmethode kommt beispielsweise die Umsetzung eines Nicotinsäure-niederalkylesters der allgemeinen Formel

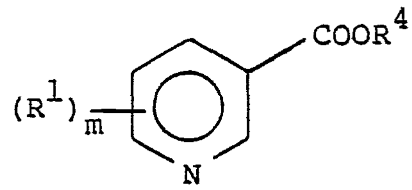

VII

worin R' und m die oben angegebenen Bedeutungen besitzen

und R⁴ einen Niederalkylrest, insbesondere Methyl, bedeutet,

mit einem Aethanolamin der oben angegebenen Formel VI in Frage. Diese Umsetzung wird zweckmässigerweise in Gegenwart von überschüssigem Aethanolamin der Formel VI als Lösungsmittel und bei erhöhten Temperaturen, vorzugsweise bei Temperaturen zwischen ca. 80°C und 160°C, durchgeführt.

Die restlichen Ausgangsmaterialien, d.h. die Nicotinsäuren der Formel II und deren reaktionsfähige Derivate, die 2-Phenoxyäthylamine der Formel III und die Phenole der Formel V, sind entweder bekannt oder können nach an sich bekannten Methoden hergestellt werden.

Die erfindungsgemässen Verbindungen, d.h. die Verbindungen der Formel I und der Säureadditionssalze, besitzen fungizide Wirkung und können dementsprechend zur Bekämpfung von Fungi in der Landwirtschaft und im Gartenbau Verwendung finden. Sie eignen sich insbesondere zur Hemmung des Wachstums oder zur Vernichtung von phytopathogenen Pilzen auf Pflanzenteilen, z.B. Blättern, Stengeln, Wurzeln, Knollen, Früchten oder Blüten, und auf Saatgut sowie im Erdboden und sind besonders wirksam bei der Bekämpfung von Septoria nodorum - (Braunfleckigkeit von Getreide), Cercosporella herpotrichoides (Halmbruchkrankheit von Getreide), Alternaria brassicae und Alternaria brassicicola - (Erreger Von Rapsschwärze) sowie Erysiphe cichoracearum (Gurkenmehltau); und von Schadpilzen der Gattungen Podosphaera, Uncinula, Puccinia, Uromyces, Hemileia, Fusarium, Rhizoctonia, Cercospora, Helminthosporium,Verticillium, Botrytis, Penicillium, Venturia, Sclerotinia und Rhynchosporium.

Einzelne Vertreter der erfindungsgemässen Verbindungen besitzen zudem eine ausgeprägte Wirkung gegen holzzerstörende Pilze, wie z.B. Coniophora puteana, Gloeophyllum trabeum, Aureobasidium pullulans und Sclerophoma pithyophila.

Die erfindungsgemässen Verbindungen zeichnen sich durch vorbeugende und kurative Wirkung sowie durch eine hohe Pflanzenverträglichkeit aus.

Die erfindungsgemässen Verbindungen wirken unter Gewächshausbedingungen bereits bei Konzentrationen von 1 mg bis 500 mg Wirkstoff pro Liter Spritzbrühe. Im Freiland werden vorzugsweise Konzentrationen von 50 g bis 1,5 kg Wirkstoff der Formel I pro Hektar und Behandlung zur Anwendung gebracht. Zur Bekämpfung von samen-oder bodenbürtigen Pilzen im Beizverfahren werden vorteilhaft Dosierungen von 0,05 bis 1,5 g Wirkstoff der Formel I pro kg Samen bzw. Erdboden eingesetzt.

Die erfindungsgemässen Verbindungen können zu verschiedenartigen Mitteln, z.B. Lösungen, Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame Menge mindestens einer Verbindung der allgemeinen Formel I, wie oben definiert, oder eines Säureadditionssalzes einer solchen Verbindung sowie Formulierungshilfsstoffe enthalten. Die Mittel enthalten zweckmässigerweise zumindest einen der folgenden Formulierungshilfsstoffe:

Feste Trägerstoffe; Lösungs-bzw. Dispersionsmittel; Tenside (Netz-und Emulgiermittel); Dispergatoren (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs-bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Al-

kohole, wie Butanol und Glykol, sowie deren Aether und Ester; Ketone, wie Aceton, Methyläthylketon, Methylisobutylketon und Cyclohexanon; und stark polare Lösungs-bzw. Dispersionsmittel, wie Dimethylformamid, N-Methylpyrrolidon und Dimethylsulfoxid, wobei solche Lösungs-bzw. Dispersionsmittel vorzugsweise Flammpunkte von mindestens 30°C und Siedepunkte von mindestens 50°C aufweisen, und Wasser. Unter den Lösungs-bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz-und Emulgiermittel) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium-und Ammoniumsalze von Ligninsulfonsäure, Natriumsalze von Maleinsäureanhydrid-Diisobutylen-Copolymeren, Natrium-und Ammoniumsalze von sulfonierten Polykondensationsprodukten aus Naphthalin und Formaldehyd, und Sulfitablaugen.

Als Dispergatoren, die sich insbesondere als Verdickungs-bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäurester und Butylhydroxytoluol; UV-Absorber, z.B. substituierte Benzophenone, Diphenylacrylnitrilsäureester und Zimtsäureester; und Deaktivatoren, z.B. Salze der Aethylendiamintetraessigsäure und Polyglykole.

Die erfindungsgemässen fungiziden Mittel können neben den Wirkstoffen der Formel I auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen, 0,0001 und 85 Gewichtsprozent an erfindungsgemässer Verbindung bzw. erfindungsgemässen Verbindungen als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 85 Gewichtsprozent, vorzugsweise 25 bis 75 Gewichtsprozent, der Verbin dung bzw. Verbingungen der Formel I. Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens eine Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden, z.B. durch Vermischen der Wirkstoffes mit festen Trägerstoffen, durch Auflösen oder Suspendieren in geeigneten Lösungs-bzw. Dispersionsmitteln, eventuell unter Verwendung von Tensiden als Netz-oder Emulgiermitteln oder von Dispergatoren, durch Verdünnen bereits vorbereiteter emulgierbarer Konzentrate mit Lösungs-bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs-bzw. Dispersionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

I. Herstellung der Wirkstoffe der Formel I:

Beispiel 1

Zu 1,9 g (8,5 mMol) 5-Bromnicotinsäurechlorid in 20 ml wasserfreiem Methylenchlorid wird unter Rühren eine Lösung von 2,4 g (8,5 mMol) N-[2-(2,4,6-Trichlorphenoxy)äthyl]-n-propylamin und 0,85 g (8,5 mMol) Triäthylamin in 10 ml Methylenchlorid getropft, und das Reaktionsgemisch wird 1 Stunde bei Raumtemperatur nachgerührt. Anschliessend wird das Gemisch mit Wasser gewaschen und über wasserfreiem Natriumsulfat getrocknet. Nach Abdestillieren des Lösungsmittels wird das zurückbleibende dunkelbraune Oel an Silicagel unter Verwendung von Chloroform als Laufmittel chromatographisch gereinigt. Man erhält auf diese Weise 3,7 g (93% der theoretischen Ausbeute) 5-Brom-N-(n-propyl)-N-[2-(2,4,6-trichlorphenoxy)äthyl]-nicotinamid als gelbliches Oel.

In analoger Weise erhält man ausgehend von:

5-Bromnicotinsäurechlorid und N-[2-(4,6-Dichlor-o-tolyloxy)äthyl]-n-propylamin das 5-Brom-N-[2-(4,6-dichlor-o-tolyloxy)äthyl]-N-(n-propyl)-nicotinamid als braunes Oel;

5-Bromnicotinsäurechlorid und N-[2-(2,4,6-Trichlorphenoxy)äthyl]-äthylamin das 5-Brom-N-äthyl-N-[2-(2,4,6-trichlorphenoxy)äthyl]-nicotinamid, Smp. 84-86°C;

5-Bromnicotinsäurechlorid und N-[2-(2,4,6-Trichlorphenoxy)äthyl]-n-butylamin das 5-Brom-N-(n-butyl)-N-[2-(2,4,6-trichlorphenoxy)äthyl] -nicotinamid als farbloses Oel;

5-Bromnicotinsäurechlorid und N-[2-(2,4,6-Trichlorphenoxy)äthyl]-n-pentylamin das 5-Brom-N-(n-pentyl)-N-[2 -(2,4,6-trichlorphenoxy)äthyl] -nicotinamid als farbloses Oel;

5-Bromnicotinsäurechlorid und N-[2-(2,4,6-Trichlorphenoxy)äthyl]-n-hexylamin das 5-Brom-N-(n-hexyl)-N-[2-(2,4,6-trichlorphenoxy)äthyl] -nicotinamid als farbloses Oel;

5-Chlornicotinsäurechlorid und N-[2-(2,4,6-Trichlorphenoxy)äthyl]-äthylamin das 5-Chlor-N-äthyl-N-[2-(2,4,6-trichlorphenoxy)äthyl]-nicotinamid, SMP. 85-88°C;

5-Chlornicotinsäurechlorid und N-[2-(2,4,6-Dichloro-tolyloxy)äthyl]-n-propylamin das 5-Chlor-N-(propyl)-N-[2-(4,6-dichlor-o-tolyloxy)äthyl] -nicotinamid als braunes Oel;

5-Chlornicotinsäurechlorid und N-[2-(2,4,6-Trichlorphenoxy)äthyl]-n-propylamin das 5-Chlor-N-(n-propyl)-N-[2-(2,4,6-trichlorphenoxy)äthyl] -nicotinamid als braunes Oel;

5-Chlornicotinsäurechlorid und N-[2-(2,4,6-Trichlorphenoxy)äthyl]-n-butylamin das 5-Chlor-N-(n-butyl)-N-[2-(2,4,6-trichlorphenoxy)äthyl] -nicotinamid als braunes Oel;

5-Chlornicotinsäurechlorid und N-[2-(2,4,6-Trichlorphenoxy)äthyl]-n-pentylamin das 5-Chlor-N-(n-pentyl)-N-[2-(2,4,6-trichlorphenoxy)äthyl]nicotinamid als braunes Oel;

5-Chlornicotinsäurechlorid und N-[2-(2,6-Dichlorphenoxy)äthyl]-n-propylamin das 5-Chlor-N-[2-(2,6-dichlorphenoxy)äthyl]-N-(n-propyl)-nicotinamid als braunes Oel;

5-Jodnicotinsäurechlorid und N-[2-(2,4,6-Trichlorphenoxy)äthyl]-äthylamin das 5-Jod-N-äthyl-N-[2-(2,4,6-trichlorphenoxy)äthyl]-nicotinamid, Smp. 92-94°C;

5-Jodnicotinsäurechlorid und N-[2-(4,6-Dichlor-o-tolyloxy)äthyl]-n-propylamin das 5-Jod-N-[2-(4,6-dichlor-o-tolyloxy)äthyl]-N-(n-propyl)-nicotinamid als braunes Oel;

5-Jodnicotinsäurechlorid und N-[2-(2,4,6-Trichlorphenoxy)äthyl]-n-propylamin das 5-Jod-N-(n-propyl)-N-[2-(2,4,6-trichlorphenoxy)äthyl] -nicotinamid als gelbliches Oel;

5-Jodnicotinsäurechlorid und N-[2-(2,6-Dichlorphenoxy)äthyl]-n-propylamin das 5-Jod-N-[2-(2,6-dichlorphenoxy)äthyl]-N-(n-propyl)-nicotinamid als braunes Oel;

5-Bromnicotinsäurechlorid und N-[2-(2,4,6-Trichlorphenoxy)äthyl]-N-(2-methoxyäthyl)-amin das 5-Brom-N-(2-methyoxyäthyl)-N-[2-(2,4,6 -trichlorphenoxy)äthyl]-nicotinamid, Smp. 85-86°C;

5,6-Dichlornicotinsäurechlorid und N-[2-(2,4,6-Trichlorphenoxy)äthyl]-n-propylamin das 5,6-Dichlor-N-(n-propyl)-N-[2-(2,4,6 -trichlorphenoxy)-äthyl]-nicotinamid, Smp. 71-72°C.

—

Beispiel 2

Eine Suspension von 22,0 g (0,1 Mol) 5-Bromnicotinsäurechlorid in 200 ml Methylenchlorid wird mit 9,2 g (0,11 Mol) Natriumhydrogencarbonat versetzt. Anschliessend tropft man unter Rühren 28,3 g N-[2-(2,4,6-Trichlorphenoxy)äthyl]-n-propylamin so zu, dass die Temperatur des Reaktionsgemisches 35°C nicht übersteigt. Man rührt das Reaktionsgemisch, eine farblose Suspension, weitere 3 Stunden bei Raumtemperatur. Danach giesst man die Suspension auf 200 ml Wasser und - schüttelt das Ganze, wobei die festen Anteile gelöst werden. Durch Zugabe von wenig gesättigter Natriumhydrogencarbonatlösung wird der pH auf ca. 7,5 eingestellt. Man extrahiert die wässrige Phase zweimal mit je 50 ml Methylenchlorid nach, trocknet die vereinigten organischen Phasen über wasserfreiem Natriumsulfat und dampft sie unter vermindertem Druck ein. Der Rückstand wird in 50 ml Toluol aufgenommen, die Lösung eingedampft und der neue Rückstand mit 100 ml Diisopropyläther versetzt und unter Rühren kristallisieren gelassen. Nach 16 Stunden wird die weisse Suspension noch 1 Stunde bei 0°C gerührt. Man nutscht ab, wäscht das Kristallpulver zweimal mit je 20 ml kaltem Diisopropyläther und erhält dabei 31,0 g farblose Kristalle, Smp. 50-52°C. Zwecks Gewinnen weiteren Produktes engt man die Mutterlauge ein, unterwirft den Rückstand einer säulenchromatographischen Reinigung an 400 g Kieselgel mit dem Laufmittel Aethylacetat/n-Hexan (1:2) (Flash-Säule), vereinigt die reinen Fraktionen und kristallisiert aus 10 ml Diispropyläther. Auf diese Weise erhält man weitere 3,4 g farblose Kristalle, Smp. 50-52°C. Die Gesamtausbeute am so hergestellten 5-Brom-N-(n-propyl)-N-[2-(2,4,6-trichlorphenoxy)äthyl]-nicotinamid beträgt 34,4 g - (73,7% der theoretischen Ausbeute).

Beispiel 3

Ein Gemisch von 3,03 g (15 mMol) 5-Bromnicotinsäure und 2,43 g (15 mMol) 1,1'-Carbonyldiimidazol wird in 50 ml Tetrahydrofuran 2,5 Stunden auf Rückflusstemperatur erhitzt. Man gibt anschliessend 3,93 g (15 mMol) N-[2-(2,6-Dichlorphenoxy)äthyl]-n-butylamin in 20 ml Tetrahydrofuran bei Raumtemperatur zu und erhitzt die resultierende Lösung weitere 3 Stunden auf Rückflusstemperatur. Danach wird das Reaktionsgemisch eingedampft, der Rückstand in Aethylacetat aufgenommen und die Lösung zweimal mit gesättigter Kochsalzlösung gewaschen. Die organische Phase wird über wasserfreiem Natriumsulfat getrocknet, eingedampft und aus

Diäthyläther/n-Hexan kristallisiert. Man erhält 2,8 g (42% der theoretischen Ausbeute) 5-Brom-N-(n-butyl)-N-[2-(2,6-dichlorphenoxy)äthyl] -nicotinamid als farblose Kristalle, Smp. 62-63°C.

In analoger Weise erhält man ausgehend von

5-Fluornicotinsäure und N-[2-(2,4,6-Trichlorphenoxy)äthyl]-n-propylamin das 5-Fluor-N-(n-propyl)-N-[2-(2,4,6-trichlorphenoxy)äthyl] -nicotinamid, Smp. 68-69°C;

5-Fluornicotinsäure und N-[2-(2,6-Dichlorphenoxy)-äthyl]-n-propylamin das 5-Fluor-N-[2-(2,6-dichlorphenoxy)äthyl]-N-(n-propyl) -nicotinamid als farbloses Oel;

Beispiel 4

Zu einer Lösung von 2,68 g (9,8 mMol) 5-Brom-N-äthyl-N-(2-hydroxyäthyl)-nicotinamid, 1,73 g (9,8 mMol) 2,4-Dichlor-6-methylphenol und 2,57 g (9,8 mMol) Triphenylphosphin in 35 ml wasserfreiem Tetrahydrofuran werden unter Rühren bei Raumtemperatur 1,98 g (9,8 mMol) Azodicarbonsäure-diisopropylester in10 ml wasserfreiem Tetrahydrofuran getropft, und das Reaktionsgemisch wird 4 Stunden nachgerührt. Danach wird das Lösungsmittel abdestilliert und das zurückbleibende Oel zweimal an Silicagel unter Verwendung von Aethylacetat als Laufmittel chromatographisch gereinigt und aus Diäthyläther kristallisiert. Man erhält dabei 2,8 g (66% der theoretischen Ausbeute) 5-Brom-N-äthyl-N-[2-(4,6-dichlor-o-tolyloxy)äthyl] -nicotinamid als beigefarbene Prismen, Smp. 73-75°C.

In analoger Weise erhält man ausgehend von:

5-Brom-N-(2-hydroxyäthyl)-N-methyl-nicotinamid und 2,6-Dichlorphenol das 5-Brom-N-[2-(2,6-dichlorphenoxy)äthyl]-N-methyl-nicotinamid, Smp. 56-57°C;

5-Brom-N-(2-hydroxyäthyl)-N-methyl-nicotinamid und 2,4,6-Trichlorphenol das 5-Brom-N-methyl-N-[2-(2,4,6-tri chlorphenoxy)äthyl]-nicotinamid als gelbliches, zähflüssiges Oel;

5-Brom-N-(2-hydroxyäthyl)-N-methyl-nicotinamid und 2,4-Dichlor-6-methylphenol das 5-Brom-N-[2-(4,6-dichlor-o-tolyloxy)äthyl]-N-methyl -nicotinamid als gelbliches, zähflüssiges Oel;

5-Brom-N-benzyl-N-(2-hydroxyäthyl)-nicotinamid und 2,4,6-Trichlorphenol das 5-Brom-N-benzyl-N-[2-(2,4,6-trichlorphenoxy)äthyl] -nicotinamid, Smp. 82-83°C;

5-Brom-N-Benzyl-N-(2-hydroxyäthyl)-nicotinamid und 2,4-Dichlor-6-methylphenol das 5-Brom-N-benzyl-N-[2-(4,6-dichlor-o-tolyloxy)äthyl] -nicotinamid, Smp. 71-72°C;

5-Brom-N-benzyl-N-(2-hydroxyäthyl)-nicotinamid und 2,6-Dichlorphenol das 5-Brom-N-benzyl-N-[2-(2,6-dichlorphenoxy)äthyl]-nicotinamid als farbloses Oel;

5-Brom-N-(2-hydroxyäthyl)-N-(n-propyl)-nicotinamid und 2,6-Dichlorphenol das 5-Brom-N-[2-(2,6-dichlorphenoxy)äthyl]-N-(n-propyl) -nicotinamid als farbloses Oel;

5-Brom-N-äthyl-N-(2-hydroxyäthyl)-nicotinamid und 2,6-Dichlorphenol das 5-Brom-N-äthyl-N-[2-(2,6-dichlorphenoxy)äthyl-nicotinamid, Smp. 78-80°C;

5-Brom-N-(n-butyl)-N-(2-hydroxyäthyl)-nicotinamid und 2,4-Dichlor-6-methylphenol das 5-Brom-N-(n-butyl)-N-[2-(4,6-dichlor-o-tolyoxy)äthyl] -nicotinamid als farbloses Oel;

5-Chlor-N-äthyl-N-(2-hydroxyäthyl)-nicotinamid und 2,4-Dichlor-6-methylphenol das 5-Chlor-N-äthyl-N-[2-(4,6-dichlor-o-tolyoxy)äthyl] -nicotinamid, Smp. 62-65°C;

5-Chlor-N-äthyl-N-(2-hydroxyäthyl)-nicotinamid und 2,6-Dichlorphenol das 5-Chlor-N-äthyl-N-[2-(2,6-dichlorphenoxy)äthyl] -nicotinamid, Smp. 65-67°C;

5-Chlor-N-benzyl-N-(2-hydroxyäthyl-nicotinamid und 2,4,6-Trichlorphenol das 5-Chlor-N-benzyl-N-[2-(2,4,6-trichlorphenoxy)äthyl] -nicotinamid, Smp. 88-89°C;

5-Chlor-N-benzyl-N-(2-hydroxyäthyl)-nicotinamid und 2,6-Dichlorphenol das 5-Chlor-N-benzyl-N-[2-(2,6-dichlorphenoxy)äthyl] -nicotinamid, Smp. 66-68°C;

5-Chlor-N-benzyl-N-(2-hydroxyäthyl)-nicotinamid und 2,4-Dichlor-6-methylphenol das 5-Chlor-N-benzyl-N-[2-(4,6-dichlor-o-tolyloxy)äthyl] -nicotinamid, Smp. 75-78°C;

5-Brom-N-(2,4-dichlorbenzyl)-N-(2-hydroxyäthyl)-nicotinamid und 2,4,6-Trichlorphenol das 5-Brom-N-(2,4-dichlorbenzyl)-N-[2-(2,4,6-trichlorphenoxy)-äthyl]-nicotinamid, Smp. 109-110°C;

5-Brom-N-(2,4-dichlorbenzyl)-N-(2-hydroxyäthyl)-nicotinamid und 2,6-Dichlorphenol das 5-Brom-N-(2,4-dichlorbenzyl)-N-[2-(2,6-dichlorphenoxy)äthyl]-nicotinamid, Smp. 160-161°C;

5-Brom-N-(3-chlorbenzyl)-N-(2-hydroxyäthyl)-nicotinamid und 2,4,6-Trichlorphenyl das 5-Brom-N-(3-chlorbenzyl)-N-[2-(2,4,6-trichlorphenoxy)äthyl]-nicotinamid als farbloses Oel;

5-Brom-N-(3-chlorbenzyl)-N-(2-hydroxyäthyl)-nicotinamid und 2,6-Dichlorphenol das 5-Brom-N-(3-chlorbenzyl)-N-[2-(2,6-dichlorphenoxy)äthyl]-nicotinamid, Smp. 105-106°C;

5-Brom-N-(2-chlorbenzyl)-N-(2-hydroxyäthyl)-nicotinamid und 2,4,6-Trichlorphenol das 5-Brom-N-(2-chlorbenzyl)-N-[2 -(2,4,6-trichlorphenoxy)-äthyl]-nicotinamid, Smp. 134-135°C.

Beispiel 5

Zu einer Lösung von 2,0 g (4,3 mMol) 5-Brom-N-(n-propyl)-N-[2-(2,4,6-trichlorphenoxy)äthyl]-nicotinamid in 10 ml Aethanol werden 10 ml 4N Salzsäure gegeben, und das Reaktionsgemisch wird ca. 16 Stunden bei Raumtemperatur stehen gelassen. Anschliessend dampft man das Lösungsmittel ab, löst das zurückbleibende leicht gelbe Oel in 10 ml Aethanol, versetzt die Lösung mit 60 ml Diäthyläther und lässt während 20 Stunden stehen. Der resultierende Niederschlag wird abgesaugt, mit Diäthyläther gewaschen und im Luftstrom getrocknet. Auf diese Weise erhält man 2,0 g (93 % der theoretischen Ausbeute) 5-Brom-N-(n-propyl)-N-[2-(2,4,6-trichlorphenoxy)äthyl]-nicotinamid-Hydrochlorid als weisse Kristalle, die sich ab 120°C zersetzen.

In analoger Weise erhält man ausgehend von 5-Fluor-N-(n-propyl)-N-[2-(2,4,6-trichlorphenoxy)-äthyl]-nicotinamid und Salzsäure das 5-Fluor-N-(n-propyl)-N-[2-(2,4,6-trichlorphenoxy)äthyl]-nicotinamid-Hydrochlorid als weisse Kristalle, die sich im Intervall von 132-138°C zersetzen.

II. Herstellung der Ausgangsmaterialien der Formel IV:

Beispiel 6

Zu einem Gemisch von 2,55 g (28 mMol) 2-Aethylaminoäthanol und 2,84 g (28 mMol) Triäthylamin in 20 ml Methylenchlorid wird unter Rühren bei Raumtemperatur eine Lösung von 4,96 g (28 mMol) 5-Chlornicotinsäurechlorid in 15 ml Methylenchlorid getropft. Das Reaktionsgemisch wird 1 Stunde bei Raumtemperatur nachgerührt und danach filtriert. Man wäscht den Niederschlag mit Methylenchlorid, destilliert das Lösungsmittel der vereinigten Filtrate ab und reinigt das zurückbleibende braune Oel an Silicagel unter Verwendung von Aethylacetat als Laufmittel chromatographisch, wobei 3,88 g (61% der theoretischen Ausbeute) 5-Chlor-N-äthyl-N-(2-hydroxyäthyl)-nicotinamid als braunes, zähflüssiges Oel erhalten werden.

In analoger Weise erhält man ausgehend von:

5-Bromnicotinsäurechlorid und 2-Methylaminoäthanol das 5-Brom-N-(2-hydroxyäthyl)-N-methyl-nicotinamid als braunes Oel;

5-Bromnicotinsäurechlorid und 2-Aethylaminoäthanol das 5-Brom-N-äthyl-N-(2-hydroxyäthyl)-nicotinamid als hellgelbes Oel;

5-Bromnicotinsäurechlorid und 2-(n-Propylamino)-äthanol das 5-Brom-N-(2-hydroxyäthyl)-N-(n-propyl)-nicotinamid als braunes Oel;

5-Chlornicotinsäurechlorid und 2-Methylaminoäthanol das 5-Chlor-N-(2-hydroxyäthyl)-N-methyl-nicotinamid;

5-Chlornicotinsäurechlorid und 2-(n-Propylamino)-äthanol das 5-Chlor-N-(2-hydroxyäthyl)-N-(n-propyl)-nicotinamid;

5-Jodnicotinsäurechlorid und 2-Methylaminoäthanol das 5-Jod-N-(2-hydroxyäthyl)-N-methyl-nicotinamid;

5-Jodnicotinsäurechlorid und 2-Aethylaminoäthanol das 5-Jod-N-äthyl-N-(2-hydroxyäthyl)-nicotinamid;

5-Jodnicotinsäurechlorid und 2-(n-Propylamino)-äthanol das 5-Jod-N-(2-hydroxyäthyl)-N-(n-propyl)-nicotinamid.

Beispiel 7

Ein Gemisch von 20,2 g (0,1 Mol) 5-Bromnicotinsäure und 16,2 g (0,1 Mol) 1,1'-Carbonyl-diimidazol in 200 ml trockenem Tetrahydrofuran wird während 2,5 Stunden auf Rückflusstemperatur erhitzt. Anschliessend gibt man zum Reaktionsgemisch 15,1 g (0,1 Mol) 2-Benzylaminoäthanol und rührt weitere 1,5 Stunden bei Rückflusstemperatur. Das Gemisch wird dann eingedampft und das zurückbleibende Oel direkt an einer Kieselgel-Flash-Säule unter Verwendung von n-Hexan/Aethylacetat (2:1) als Laufmittel

chromatographisch gereinigt. Man erhält 22,7 g - (68% der theoretischen Ausbeute) 5-Brom-N-benzyl-N-(2-hydroxyäthyl)-nicotinamid als farbloses Oel.

In analoger Weise erhält man ausgehend von:

5-Bromnicotinsäure, 1,1'-Carbonyl-diimidazol und 2-(n-Butylamino)äthanol das 5-Brom-N-(n-butyl)-N-(2-hydroxyäthyl)-nicotinamid als farbloses Oel;

5-Chlornicotinsäure, 1,1'-Carbonyl-diimidazol und 2-Benzylaminoäthanol das 5-Chlor-N-benzyl-N-(2-hydroxyäthyl)-nicotinamid als farbloses Oel;

5-Bromnicotinsäure, 1,1'-Carbonyl-diimidazol und 2-(2-Chlorbenzyl)aminoäthanol das 5-Brom-N-(2-chlorbenzyl)-N-(2-hydroxyäthyl)-nicotinamid als farbloses Oel;

5-Bromnicotinsäure, 1,1'-Carbonyl-diimidazol und 2-(3-Chlorbenzyl)aminoäthanol das 5-Brom-N-(3-chlorbenzyl)-N-(2-hydroxyäthyl)-nicotinamid als farbloses Oel;

5-Bromnicotinsäure, 1,1'-Carbonyl-diimidazol und 2-(2,4-Dichlorbenzyl)aminoäthanol das 5-Brom-N-(2,4-dichlorbenzyl)-N-(2-hydroxyäthyl)-nicotinamid als farbloses Oel.

III> Formulierungsbeispiele:

Beispiel 8

(a) Flüssige Wirkstoffe

1. Emulgierbares Konzentrat:

| | | g/l |
|---|---|---|
| Wirkstoff der Formel I | | 250 |
| Nonylphenol-(10)-äthoxylat | | 50-70 |
| Calcium-dodecylbenzolsulfonat | | 25 |
| Gemisch von $C_{10}$-Alkylbenzolen | ad | 1 l |

Die Komponenten werden miteinander vermischt, bis eine klare Lösung entsteht.

2. Spritzpulver:

| | Gewichtsteile |
|---|---|
| Wirkstoff der Formel I | 25 |
| Hydratisierte Kieselsäure | 25 |
| Natrium-lignosulfonat | 10 |
| Natrium-laurylsulfat | 2 |
| Calciumcarbonat | 38 |
| | 100 |

Der flüssige oder geschmolzene Wirkstoff wird auf die Kieselsäure aufgezogen, die übrigen Komponenten zugemischt und das Ganze in einer geeigneten Mühle feingemahlen.

(b) Feste Wirkstoffe

Spritzpulver:

| | Gewichtsteile |
|---|---|
| Wirkstoff der Formel I | 50 |
| Hydratisierte Kieselsäure | 5 |
| Natrium-laurylsulfat | 1 |
| Natrium-lignosulfonat | 2 |
| Kaolin und/oder Tonerde | 42 |
| | 100 |

Die Komponenten werden miteinander vermischt, und das Ganze wird dann in einer geeigneten Mühle feingemahlen.

**Ansprüche**

1. Verbindungen der allgemeinen Formel

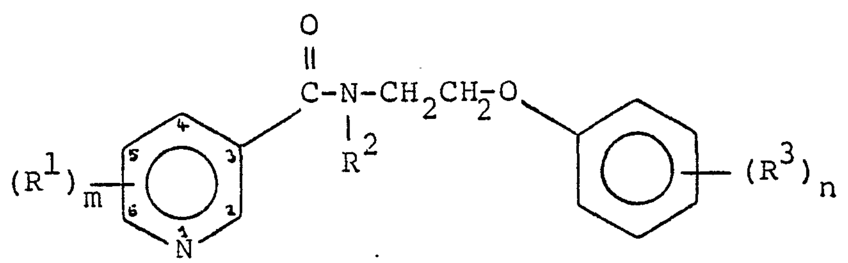

I

worin

$R^1$ Halogen in der 5-und/oder 6-Stellung der Pyridylgruppe,

$R^2$ $C_{1-6}$-Alkyl, $C_{1-6}$-Alkoxy-$C_{1-4}$-alkyl, Cyano-$C_{1-4}$-alkyl, $C_{3-6}$-Alkinyl oder gegebenenfalls im Benzolkern mono-, di-oder trisubstituiertes Benzyl, wobei die gegebenenfalls vorhandenen Substituenten 1 bis 3 Halogenatome und/oder 1 oder 2 $C_{1-3}$ -Alkylgruppen und/oder 1 oder 2 $C_{1-3}$-Alkoxygruppen und/oder eine Trifluormethylgruppe und/oder eine Nitrogruppe sind,

$R^3$ Halogen, $C_{1-3}$-Alkyl oder Nitro,

m 1 oder 2

und

n 1, 2 oder 3 bedeuten,

und Säureadditionssalze dieser Verbindungen.

2. Verbindungen nach Anspruch 1, worin $R^1$ Fluor, Chlor oder Brom bedeutet.

3. Verbindungen nach Anspruch 1 oder 2, worin m 1 bedeutet.

4. Verbindungen nach Anspruch 3, worin das Halogenatom $R^1$ in 5-Stellung ist.

5. Verbindungen nach einem der Ansprüche 1 bis 4, worin $R^2$ Aethyl, n-Propyl, oder n-Butyl bedeutet.

6. Verbindungen nach einem der Ansprüche 1 bis 5, worin $R^3$ Chlor oder Methyl bedeutet.

7. Verbindungen nach einem der Ansprüche 1 bis 6, worin n 2 oder 3 bedeutet.

8. Verbindungen nach Ansprüchen 6 und 7, worin die mit $(R^3)n$ substituierte Phenylgruppe 2,4,6-Trichlorphenyl, 2,4-Dichlor-6-methylphenyl oder 2,6-Dichlorphenyl bedeutet.

9. Eine Verbindung nach Anspruch 1, ausgewählt aus:

5-Fluor-N-(n-propyl)-N-[2-(2,4,6-trichlorphenoxy)-äthyl]-nicotinamid,

5-Chlor-N-(n-propyl)-N-[2-(2,4,6-trichlorphenoxy)-äthyl]-nicotinamid,

5-Brom-N-(n-propyl)-N-[2-(2,4,6-trichlorphenoxy)-äthyl]-nicotinamid,

5-Brom-N-[2-(2,6-dichlorphenoxy)äthyl]-N-(n-propyl)-nicotinamid und

5-Brom-N-[2-(4,6-dichlor-o-tolyloxy)äthyl]-N-(n-propyl)-nicotinamid.

10. Eine Verbindung nach Anspruch 1, ausgewählt aus:

5-Brom-N-äthyl-N-[2-(2,4,6-trichlorphenoxy)äthyl]-nicotinamid,

5-Brom-N-(n-butyl)-N-[2-(2,4,6-trichlorphenoxy)-äthyl]-(nicotinamid,

5-Brom-N-(n-pentyl)-N-[2-(2,4,6-trichlorphenoxy)-äthyl]-nicotinamid,

5-Brom-N-(n-hexyl)-N-[2-(2,4,6-trichlorphenoxy)-äthyl]-nicotinamid,

5-Chlor-N-äthyl-N-[2-(2,4,6-trichlorphenoxy)äthyl]-nicotinamid,

5-Chlor-N-(n-propyl)-N-[2-(4,6-dichlor-o-tolyoxy)-äthyl]-nicotinamid,

5-Chlor-N-(n-butyl)-N-[2-(2,4,6-trichlorphenoxy)-äthyl]-nicotinamid,

5-Chlor-N-(n-pentyl)-N-[2-(2,4,6-trichlorphenoxy)-äthyl]-nicotinamid,

5-Chlor-N-[2-(2,6-dichlorphenoxy)äthyl]-N-(n-propyl)-nicotinamid,

5-Jod-N-äthyl-N-[2-(2,4,6-trichlorphenoxy)äthyl]-nicotinamid.

5-Jod-N-[2-(4,6-dichlor-o-tolyloxy)äthyl]-N-(n-propyl)-nicotinamid,

5-Jod-N-(n-propyl)-N-[2-(2,4,6-trichlorphenoxy)-äthyl]-nicotinamid,

5-Jod-N-[2-(2,6-dichlorphenoxy)äthyl]-N-(n-propyl)-nicotinamid,

5-Brom-N-(n-butyl)-N-[2-(2,6-dichlorphenoxy)äthyl]-nicotinamid,

5-Fluor-N-[2-(2,6-dichlorphenoxy)äthyl]-N-(n-propyl)-nicotinamid,

5-Brom-N-äthyl-N-[2-(4,6-dichlor-o-tolyloxy)äthyl]-nicotinamid,

5-Brom-N-[2-(2,6-dichlorphenoxy)äthyl]-N-methyl-nicotinamid,

5-Brom-N-methyl-N-[2-(2,4,6-trichlorphenoxy)äthyl]-nicotinamid,

5-Brom-N-[2-(4,6-dichlor-o-tolyloxy)äthyl]-N-methyl-nicotinamid,

5-Brom-N-benzyl-N-[2-(2,4,6-trichlorphenoxy)äthyl]-nicotinamid,

5-Brom-N-benzyl-N-[2-(4,6-dichlor-o-tolyloxy)äthyl]-nicotinamid,

5-Brom-N-benzyl-N-[2-(2,6-dichlorphenoxy)äthyl]-nicotinamid,

5-Brom-N-äthyl-N-[2-(2,6-dichlorphenoxy)äthyl]-nicotinamid,

5-Brom-N-(n-butyl)-N-[2-(4,6-dichlor-o-tolyloxy)-äthyl]-nicotinamid,

5-Chlor-N-äthyl-N-[2-(4,6-dichlor-o-tolyloxy)äthyl]-nicotinamid,

5-Chlor-N-äthyl-N-[2-(2,6-dichlorphenoxy)äthyl]-nicotinamid,

5-Chlor-N-benzyl-N-[2-(2,4,6-trichlorphenoxy)äthyl]-nicotinamid,

5-Chlor-N-benzyl-N-[2-(2,6-dichlorphenoxy)äthyl]-nicotinamid,

5-Chlor-N-benzyl-N-[2-(4,6-dichlor-o-tolyloxy)äthyl]-nicotinamid und

5-Brom-N-(2,4-dichlorbenzyl)-N-[2-(2,4,6-trichlorphenoxy-äthyl]-nicotinamid.

11. Eine Verbindung nach Anspruch 1, ausgewählt aus:

5-Brom-N-(2-methoxyäthyl)-N-[2-(2,4,6-trichlorphenoxy)äthyl]-nicotinamid,

5,6-Dichlor-N-(n-propyl)-N-[2-(2,4,6-trichlorphenoxy)äthyl]-nicotinamid,

5-Brom-N-(2,4-dichlorbenzyl)-N-[2-(2,6-dichlorphenoxy)äthyl]-nicotinamid,

5-Brom-N-(3-chlorbenzyl)-N-[2-(2,4,6-trichlorphenoxy)äthyl]-nicotinamid,

5-Brom-N-(3-chlorbenzyl)-N-[2-(2,6-dichlorphenoxy)äthyl]-nicotinamid,

5-Brom-N-(2-chlorbenzyl)-N-[2-(2,4,6-trichlorphenoxy)äthyl]-nicotinamid,

5-Brom-N-(n-propyl)-N-[2-(2,4,6-trichlorphenoxy)-äthyl]-nicotinamid-Hydrochlorid und

5-Fluor-N-(n-propyl)-N-[2-(2,4,6-trichlorphenoxy)-äthyl]-nicotinamid-Hydrochlorid.

12. Fungizides Mittel, dadurch gekennzeichnet, dass es eine wirksame Menge mindestens einer Verbindung der allgemeinen Formel

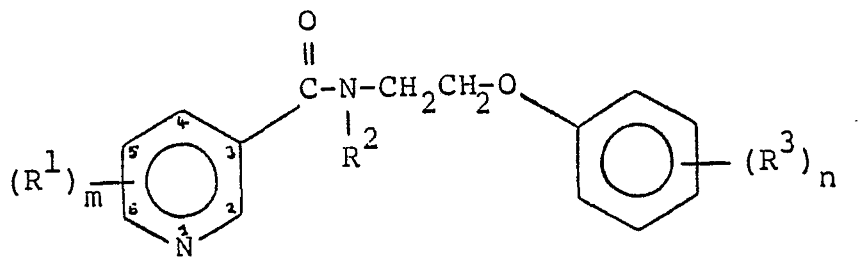

I

worin

$R^1$ Halogen in der 5-und/oder 6-Stellung der Pyridylgruppe

$R^2$ $C_{1-6}$-Alkyl, $C_{1-6}$ -Alkoxy-$C_{1-4}$-alkyl, Cyano-$C_{1-4}$-alkyl, C $_{3-6}$-Alkinyl oder gegebenenfalls im Benzolkern mono-, di-oder trisubstituiertes Benzyl, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Trifluormethylgruppe und/oder eine Nitrogruppe sind,

$R^3$ Halogen, $C_{1-3}$-Alkyl oder Nitro,

m 1 oder 2

und

n 1, 2 oder 3 bedeuten,

oder eines Säureadditionssalzes einer solchen Verbindung sowie Formulierungshilfsstoffe enthält.

13. Fungizides Mittel nach Anspruch 12, dadurch gekennzeichnet, dass es eine wirksame Menge mindestens einer aus der Gruppe

5-Fluor-N-(n-propyl)-N-[2-(2,4,6-trichlorphenoxy)-äthyl]-nicotinamid,

5-Chlor-N-(n-propyl)-N-[2-(2,4,6-trichlorphenoxy)-äthyl]-nicotinamid,

5-Brom-N-(n-propyl)-N-[2-(2,4,6-trichlorphenoxy)-äthyl]-nicotinamid,

5-Brom-N-[2-(2,6-dichlorphenoxy)äthyl]-N-(n-propyl)-nicotinamid und

5-Brom-N-[2-(4,6-dichlor-o-tolyloxy)äthyl]-N-(n-propyl)-nicotinamid

ausgewählten Verbindung sowie Formulierungshilfsstoffe enthält.

14. Verfahren zur Herstellung von Verbindungen der allgemeinen Formel

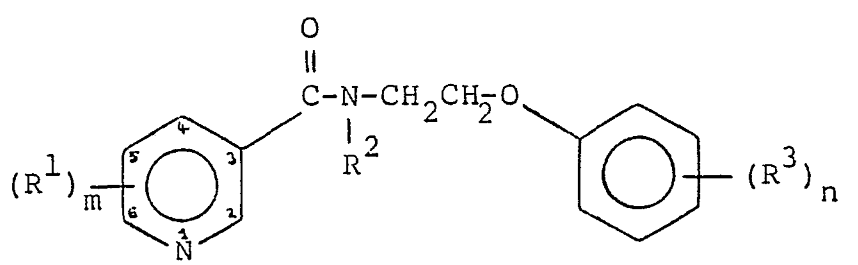

I

worin

$R^1$ Halogen in der 5-und/oder 6-Stellung der Pyridylgruppe,

$R^2$ $C_{1-6}$-Alkyl, $C_{1-6}$ -Alkoxy-$C_{1-4}$-alkyl, Cyano-$C_{1-4}$-alkyl, C $_{3-6}$-Alkinyl oder gegebenenfalls im Benzolkern mono-, di-oder trisubstituiertes Benzyl, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Trifluormethylgruppe und/oder eine Nitrogruppe sind,

$R^3$ Halogen, $C_{1-3}$-Alkyl oder Nitro,

m 1 oder 2

und

n 1, 2 oder 3 bedeuten,

und von Säureadditionssalzen dieser Verbindungen, dadurch gekennzeichnet, dass man

a) ein reaktionsfähiges Derivat einer Nicotinsäure der allgemeinen Formel

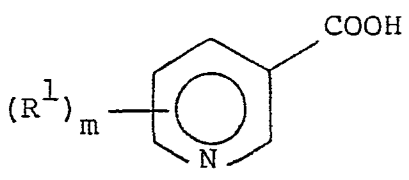

II

worin $R^1$ und m die oben angegebenen Bedeutungen besitzen,

mit einem 2-Phenoxyäthylamin der allgemeinen Formel

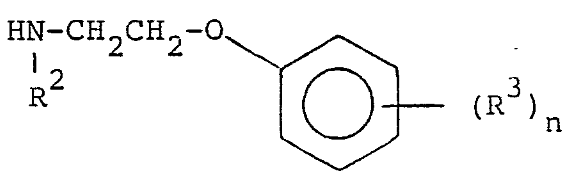

III

worin $R^2$, $R^3$ und n die oben angegebenen Bedeutungen besitzen,

umsetzt oder

b) ein N-(2-Hydroxyäthyl)-nicotinamid der allgemeinen Formel

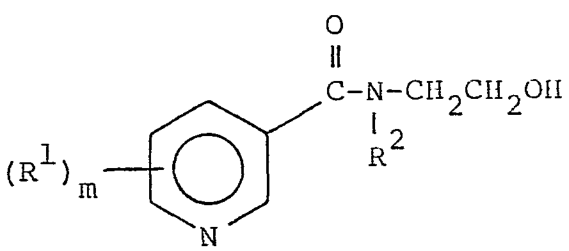

IV

worin $R^1$, $R^2$ und m die oben angegebenen Bedeutungen besitzen,

mit einem Phenol der allgemeinen Formel

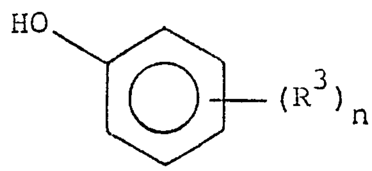

V

worin $R^3$ und n die oben angegebenen Bedeutungen besitzen,

umsetzt,

und gewünschtenfalls eine so erhaltene Verbindung der Formel I durch Umsetzung mit einer Säure in das entsprechende Säureadditionssalz überführt.

15. Verfahren zur Bekämpfung von Fungi in der Landwirtschaft und im Gartenbau, dadurch gekennzeichnet, dass man das zu schützende Gut mit einer wirksamen Menge einer Verbindung gemäss einem der Ansprüche 1 bis 11 bzw. eines Mittels gemäss Anspruch 12 oder 13 behandelt.

16. Verwendung einer Verbindung gemäss einem der Ansprüche 1 bis 11 bzw. eines Mittels gemäss Anspruch 12 oder 13 zur Bekämpfung von Fungi in der Landwirtschaft und im Gartenbau.

Patentanspruch(üche) für den Vertragsstaat : AT

1. Fungizides Mittel, dadurch gekennzeichnet, dass es eine wirksame Menge mindestens einer Verbindung der allgemeinen Formel

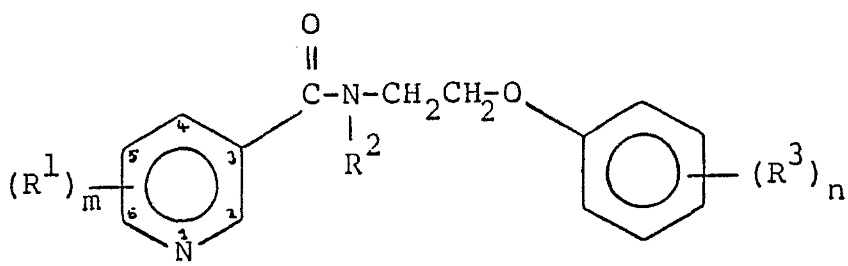

I

worin

$R^1$ Halogen in der 5-und/oder 6-Stellung der Pyridylgruppe,

$R^2$ $C_{1-6}$-Alkyl, $C_{1-6}$-Alkoxy-$C_{1-4}$-alkyl, Cyano-$C_{1-4}$-alkyl, $C_{3-6}$-Alkinyl oder gegebenenfalls im Benzolkern mono-, di-oder trisubstituiertes Benzyl, wobei die gegebenenfalls vorhandenen Substituenten 1 bis 3 Halogenatome und/oder 1 oder 2 $C_{1-3}$ -Alkylgruppen und/oder 1 oder 2 $C_{1-3}$-Alkoxygruppen und/oder eine Trifluormethylgruppe und/oder eine Nitrogruppe sind,

$R^3$ Halogen, $C_{1-3}$-Alkyl oder Nitro,

m 1 oder 2

und

n 1, 2 oder 3 bedeuten,

oder eines Säureadditionssalzes einer solchen Verbindung sowie Formulierungshilfsstoffe enthält.

2. Fungizides Mittel nach Anspruch 1, worin $R^1$ Fluor, Chlor oder Brom bedeutet.

3. Fungizides Mittel nach Anspruch 1 oder 2, worin m 1 bedeutet.

4. Fungizides Mittel nach Anspruch 3, worin das Halogenatom $R^1$ in 5-Stellung ist.

5. Fungizides Mittel nach einem der Ansprüche 1 bis 4, worin $R^2$ Aethyl, n-Propyl oder n-Butyl bedeutet.

6. Fungizides Mittel nach einem der Ansprüche 1 bis 5, worin $R^3$ Chlor oder Methyl bedeutet.

7. Fungizides Mittel nach einem der Ansprüche 1 bis 6, worin n 2 oder 3 bedeutet.

8. Fungizides Mittel nach Ansprüchen 6 und 7,

worin die mit ($R^3$)n substituierte Phenylgruppe 2,4,6-Trichlorphenyl, 2,4-Dichlor-6-methylphenyl oder 2,6-Dichlorphenyl bedeutet.

9. Fungizides Mittel nach Anspruch 1, dadurch gekennzeichnet, dass es eine wirksame Menge mindestens einer aus der Gruppe

5-Fluor-N-(n-propyl)-N-[2-(2,4,6-trichlorphenoxy)äthyl]-nicotinamid,

5-Chlor-N-(n-propyl)-N-[2-(2,4,6-trichlorphenoxy)äthyl]-nicotinamid,

5-Brom-N-(n-propyl)-N-[2-(2,4,6-trichlorphenoxy)äthyl]-nicotinamid,

5-Brom-N-[2-(2,6-dichlorphenoxy)äthyl]-N-(n-propyl)-nicotinamid und

5-Brom-N-[2-(4,6-dichlor-o-tolyloxy)äthyl]-N-(n-propyl)-nicotinamid.

ausgewählten Verbindung sowie Formulierungshilfsstoffe enthält.

10. Fungizides Mittel nach Anspruch 1, dadurch gekennzeichnet, dass es eine wirksame Menge mindestens einer aus der Gruppe

5-Brom-N-äthyl-N-[2-(2,4,6-trichlorphenoxy)-äthyl]-nicotinamid,

5-Brom-N-(n-butyl)-N-[2-(2,4,6-trichlorphenoxy)äthyl]-nicotinamid,

5-Brom-N-(n-pentyl)-N-[2-(2,4,6-trichlorphenoxy)äthyl]-nicotinamid,

5-Brom-N-(n-hexyl)-N-[2-(2,4,6-trichlorphenoxy)äthyl]-nicotinamid,

5-Chlor-N-äthyl-N-[2-(2,4,6-trichlorphenoxy)-äthyl]-nicotinamid,

5-Chlor-N-(n-propyl)-N-[2-(4,6-dichlor-o-tolyloxy)äthyl]-nicotinamid,

5-Chlor-N-(n-butyl)-N-[2-(2,4,6-trichlorphenoxy)äthyl]-nicotinamid,

5-Chlor-N-(n-pentyl-N-[2-(2,4,6-trichlorphenoxy)äthyl]-nicotinamid,

5-Chlor-N-[2-(2,6-dichlorphenoxy)äthyl]-N-(n-propyl)-nicotinamid,

5-Jod-N-äthyl-N-[2-(2,4,6-trichlorphenoxy)-äthyl]-nicotinamid.

5-Jod-N-[2-(4,6-dichlor-o-tolyloxy)äthyl]-N-(n-propyl)-nicotinamid,

5-Jod-N-(n-propyl)-N-[2-(2,4,6-trichlorphenoxy)äthyl]-nicotinamid,

5-Jod-N-[2-(2,6-dichlorphenoxy)äthyl]-N-(n-propyl)-nicotinamid,

5-Brom-N-(n-butyl)-N-[2-(2,6-dichlorphenoxy)-äthyl]-nicotinamid,

5-Fluor-N-[2-(2,6-dichlorphenoxy)äthyl]-N-(n-propyl)-nicotinamid,

5-Brom-N-äthyl-N-[2-(4,6-dichlor-o-tolyloxy)-äthyl]-nicotinamid,

5-Brom-N-[2-(2,6-dichlorphenoxy)äthyl]-N-methyl-nicotinamid,

5-Brom-N-methyl-N-[2-(2,4,6-trichlorphenoxy)-äthyl]-nicotinamid,

5-Brom-N-[2-(4,6-dichlor-o-tolyloxy)äthyl]-N-methyl-nicotinamid,

5-Brom-N-benzyl-N-[2-(2,4,6-trichlorphenoxy)-äthyl]-nicotinamid,

5-Brom-N-benzyl-N-[2-(4,6-dichlor-o-tolyloxy)-äthyl]-nicotinamid,

5-Brom-N-benzyl-N-[2-(2,6-dichlorphenoxy)-äthyl]-nicotinamid,

5-Brom-N-äthyl-N-[2-(2,6-dichlorphenoxy)-äthyl]-nicotinamid,

5-Brom-N-(n-butyl)-N-[2-(4,6-dichlor-o-tolyloxy)äthyl]-nicotinamid,

5-Chlor-N-äthyl-N-[2-(4,6-dichlor-o-tolyloxy)-äthyl]-nicotinamid,

5-Chlor-N-äthyl-N-[2-(2,6-dichlorphenoxy)-äthyl]-nicotinamid,

5-Chlor-N-benzyl-N-[2-(2,4,6-trichlorphenoxy)-äthyl]-nicotinamid,

5-Chlor-N-benzyl-N-[2-(2,6-dichlorphenoxy)-äthyl]-nicotinamid,

5-Chlor-N-benzyl-N-[2-(4,6-dichlor-o-tolyloxy)-äthyl]-nicotinamid und

5-Brom-N-(2,4-dichlorbenzyl)-N-[2-(2,4,6-trichlorphenoxy)äthyl]-nicotinamid

ausgewählten Verbindung sowie Formulierungshilfsstoffe enthält.

11. Fungizides Mittel nach Anspruch 1, dadurch gekennzeichnet, dass es eine wirksame Menge mindestens einer aus der Gruppe

5-Brom-N-(2-methoxyäthyl)-N-[2-(2,4,6-trichlorphenoxy)äthyl]-nicotinsäure,

5,6-Dichlor-N-(n-propyl)-N-[2-(2,4,6-trichlorphenoxy)äthyl]-nicotinamid,

5-Brom-N-(2,4-dichlorbenzyl)-N-[2-(2,6-dichlorphenoxy)äthyl]-nicotinamid,

5-Brom-N-(3-chlorbenzyl)-N-[2-(2,4,6-trichlorphenoxy)äthyl]-nicotinamid,

5-Brom-N-(3-chlorbenzyl)-N-[2-(2,6-dichlorphenoxy)äthyl]-nicotinamid,

5-Brom-N-(2-chlorbenzyl-N-[2-(2,4,6-trichlorphenoxy)äthyl]-nicotinamid,

5-Brom-N-(n-propyl)-N-[2-(2,4,6-trichlorphenoxy)äthyl]-nicotinamid-Hydrochlorid und

5-Fluor-N-(n-propyl)-N-[2-(2,4,6-trichlorphenoxy)äthyl]-nicotinamid-Hydrochlorid.

ausgewählten Verbindung sowie Formulierungshilfsstoffe enthält.

12. Verfahren zur Herstellung von Verbindungen der allgemeinen Formel

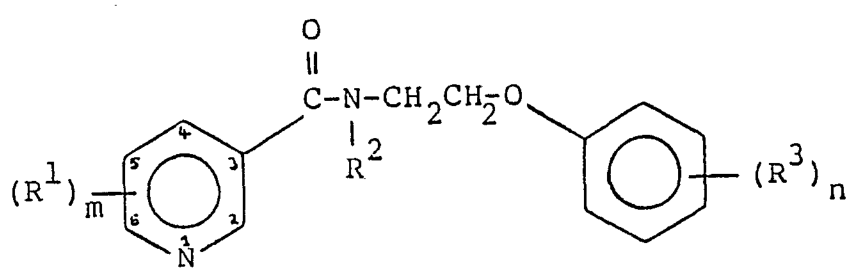

I

worin

$R^1$ Halogen in der 5-und/oder 6-Stellung der Pyridylgruppe

$R^2$ $C_{1-6}$-Alkyl, $C_{1-6}$ -Alkoxy-$C_{1-4}$-alkyl, Cyano-$C_{1-4}$-alkyl, $C_{3-6}$-Alkinyl oder gegebenenfalls im Benzolkern mono-, di-oder trisubstituiertes Benzyl, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Trifluormethylgruppe und/oder eine Nitrogruppe sind,

$R^3$ Halogen, $C_{1-3}$-Alkyl oder Nitro,

m 1 oder 2

und

n 1, 2 oder 3 bedeuten,

und von Säureadditonssalzen dieser Verbindungen, dadurch gekennzeichnet, dass man

a) ein reaktionsfähiges Derivat einer Nicotinsäure der allgemeinen Formel

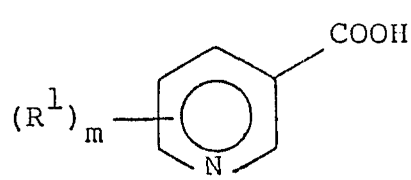

II

worin $R^1$ und m die oben angegebenen Bedeutungen besitzen,

mit einem 2-Phenoxyäthylamin der allgemeinen Formel

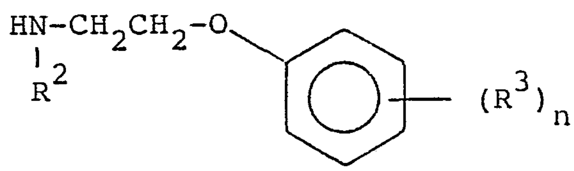

III

worin $R^2$, $R^3$ und n die oben angegebenen Bedeutungen besitzen,

umsetzt oder

b) ein N-(2-Hydroxyäthyl)-nicotinamid der allgemeinen Formel

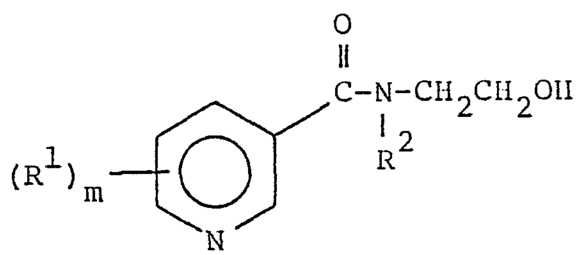

IV

worin $R^1$, $R^2$ und m die oben angegebenen Bedeutungen besitzen,

mit einem Phenol der allgemeinen Formel

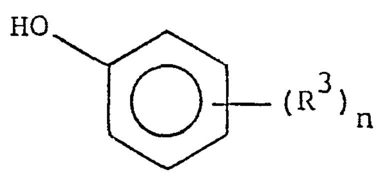

V

worin $R^3$ und n die oben angegebenen Bedeutungen besitzen,

umsetzt,

und gewünschtenfalls eine so erhaltene Verbindung der Formel I durch Umsetzung mit einer Säure in das entsprechende Säureadditionssalz überführt.

13. Verfahren zur Herstellung eines fungiziden Mittels, dadurch gekennzeichnet, dass man mindestens eine der in Anspruch 1 genannten Verbindungen mit Formulierungshilfsstoffen vermischt.

14. Verfahren zur Bekämpfung von Fungi in der Landwirtschaft und im Gartenbau, dadurch gekennzeichnet, dass man das zu schützende Gut mit einer wirksamen Menge einer Verbindung bzw. eines Mittels gemäss einem der Ansprüche 1 bis 10 behandelt.

15. Verfahren zur Bekämpfung von Fungi in der Landwirtschaft und im Gartenbau, dadurch gekennzeichnet, dass man das zu schützende

Gut mit einer wirksamen Menge einer Verbindung bzw. eines Mittels gemäss Anspruch 11 behandelt.

16. Verwendung einer Verbindung bzw. eines Mittels gemäss einem der Ansprüche 1 bis 10 zur Bekämpfung von Fungi in der Landwirtschaft und im Gartenbau.

17. Verwendung einer Verbindung bzw. eines Mittels gemäss Anspruch 11 zur Bekämpfung von Fungi in der Landwirtschaft und im Gartenbau.